# EUROPEAN PATENT SPECIFICATION

(11) **EP 3 496 703 B1**
(45) Date of publication and mention of the grant of the patent: **12.05.2021**
(21) Application number: 17752468.3
(22) Date of filing: 11.08.2017
(51) Int. Cl.: A61K 9/08, A61K 31/365, A61K 47/10, A61K 47/14, A61K 47/32

(54) **MOXIDECTIN TOPICAL LIQUID FORMULATIONS**
TOPISCHE MOXIDECTIN-FLÜSSIGFORMULIERUNGEN
FORMULATIONS LIQUIDES TOPIQUES À BASE DE LA MOXIDECTINE

(30) Priority: 12.08.2016 GB 201613842
(43) Date of publication of application: 19.06.2019
(73) Proprietor: Norbrook Laboratories Limited, Newry, County Down BT35 6PU (GB)
(72) Inventor: REYNOLDS, Louise, Banbridge Down BT32 3RG (GB)
(74) Representative: HGF
(86) International application number: PCT/GB2017/052374
(87) International publication number: WO 2018/029487

(56) References cited:
- EP-A1- 1 136 081
- WO-A1-2009/018198
- WO-A1-2014/098619
- AU-A4- 2006 100 661
- US-B1- 6 514 951

## Description

### Technical Field

The present invention relates to topical liquid formulations comprising moxidectin, a method for their production and their use in the treatment and prevention of endoparasites and ectoparasites in animals.

### Background Art

Milbemycins are compounds used for treating or controlling endo- and ectoparasite infestations. These compounds are characterized by a 16-membered macrocyclic lactone ring. Examples of milbemycins include, but are not limited to, lepimectin, milbemectin, milbemycin D, milbemycin oxime, moxidectin and nemadectin, see EP1505069, US4916154, US3984564, US4916154 and US3950360.

The use of topical liquid formulations for treating parasite infestations in veterinary medicine has increased considerably in the last few years. This growth is attributed in great part to the ease of use of these formulations, see US8653128. CYDECTIN®, a moxidectin pour-on composition, is an example of such a widely employed topical liquid formulation, see EP0432494 and US6514951. However, there exists a continuing need for new and improved moxidectin veterinary compositions which can be used for preventing and treating endo- and ectoparasites.

### Summary of the Invention

The present invention relates to liquid topical compositions comprising moxidectin, isopropyl alcohol, a thickener and a pharmaceutically acceptable oil. The compositions of the invention are useful for treating and preventing endo- and ectoparasite infestations in animals. These compositions contain a relatively low level of impurities and are significantly more stable than other moxidectin liquid compositions known in the art. They are also surprisingly stable at warm temperatures and under high humidity conditions.

The topical nature of the compositions of the invention confers on them a significant advantage, as they are easier to administer than comparable solid and injectable forms. This last trait makes the compositions of the invention particularly useful for treating mammals and, especially, domestic and farm animals.

Therefore, in a first aspect, the present invention is directed to a non-aqueous topical composition comprising:
i) moxidectin or its pharmaceutically acceptable salts,
ii) from 1% to 50% (w/v) of isopropyl alcohol,
iii) from 1% to 20% (w/v) of at least one thickener, and
iv) a pharmaceutically acceptable oil.

Said composition is characterized by being in liquid form and stable at relatively high temperature and humidity conditions. The composition of the invention is also characterized by a low level of impurities.

In another aspect, the present invention refers to a liquid topical composition according to the first aspect of the invention for use as a medicament.

In a further aspect, the present invention is directed to a liquid topical composition according to the first aspect of the invention for use in the treatment or prevention of endoparasites, ectoparasites or both in an animal subject. Alternatively, the present invention relates to the use of a liquid topical composition according to the first aspect of the invention in the manufacture of a medicament for the treatment or prevention of endoparasites, ectoparasites or both in an animal subject. The present invention refers also to a method of treating or preventing an endoparasite or ectoparasite infestation in an animal subject which comprises administering to said subject a therapeutically effective amount of the liquid topical composition of the first aspect of the invention.

In an additional aspect, the present invention relates to a process of making the liquid compositions according to the first aspect of the invention.

### Detailed Description of the Invention

### 1. Definitions

The term "animal subject" as used herein, refers to a multicellular eukaryotic organism, such as a non-human primate (e.g. chimpanzees, apes, monkey species), farm animal (e.g. cattle, sheep, pigs, goats, horses, poultry), domestic mammal (e.g. dogs, cats) or laboratory animal (e.g. mice, rats, guinea pigs). The term does not denote any particular age or sex. The term "animal subject" as used herein does not include human beings.

The term "excipient" as used herein, means any component, other than an active agent, intentionally added to a formulation. Exemplary excipients include, but are not limited to, acids (e.g. citric acid, tartaric acid), alcohols, antioxidants, bicarbonate salts (e.g. sodium, potassium), binders, bittering agents, colorants, disintegrants, fillers, foaming agents, lubricants, plasticizers, pore-forming agents, sodium chloride, solvents, spreading agents and stabilizers. Some excipients can serve multiple purposes. For instance, polybutene can be used as a solvent and/or antioxidant.

The term "moxidectin" as used herein, refers to (2aE,4E,5'R,6R,6'S, 8E,11R,13S,15S,17aR,20R,20aR,20bS)-6'-((E)-1,3-dimethyl-1-butenyl)- 5',6,6',7,10,11,14, 15,17a,20,20a,20b-dodecahydro-20,20b-dihydroxy-5',6,8,19-tetramethylspiro (11,15-methano-2H,13H,17H-furo(4,3,2-pq)(2,6)benzodioxacyclooctadecin-13,2'-(2H) pyran)-4',17(3'H)-dione 4'-(E)-(O-methyloxime), CAS [113507-06-5], also known as milbemycin B, a compound of molecular formula C₃₇H₅₃Cl₂NO₈ and structural formula:

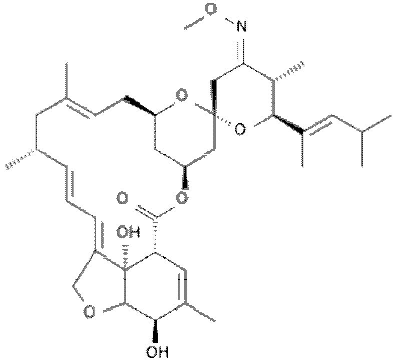

The terms "number average molecular weight" and Mₙ as used herein, refer to the statistical average molecular weight of all the polymer chains in a sample, and is defined by the formula Mₙ = (∑NᵢMᵢ)/∑Nᵢ wherein Mᵢ is the molecular weight of a chain and Nᵢ is the number of chains of that particular molecular weight.

The term "oil" as used herein, refers to any neutral, nonpolar carbon-based substance that is in liquid state at room temperature and is both hydrophobic and lipophilic. An oil can be saturated, partially or fully unsaturated. It can also be synthetic or naturally occurring (e.g. derived from vegetable, animal or marine sources). Examples of partially or fully unsaturated oils include, but are not limited to, naturally occurring oils such as castor oil, safflower oil, cottonseed oil, corn oil, olive oil, cod liver oil, almond oil, avocado oil, palm oil, sesame oil, peanut oil and soybean oil. Examples of fully saturated oils include, but are not limited to, esters of medium to large chain fatty acids (e.g. C₆-C₂₄ fatty acid triglycerides). Mixtures of fatty acids may be split from the source natural oil (e.g. coconut oil, palm kernel oil, babassu oil) and refined. Saturated C₈-C₁₂ fatty acid medium chain triglycerides (MCT) are particularly useful oils for carrying out the invention. These saturated oils comprise typically capric acid (i.e. 20-45%) and caprylic acid (i.e. 45-80%). Fully saturated oils include, but are not limited to, saturated coconut oil (i.e. a mixture of lauric, myristic, palmitic, capric and caprylic acids) and its derivatives (e.g. NEOBEE® M-5 oil, Stepan Specialty Products, LLC, Maywood, NJ, US). Examples of synthetic oils include, but are not limited to, triglycerides and propylene glycol di-esters of C₆-C₂₄ fatty acids. Said compounds comprise carboxylic acids groups having from 6 to 24 carbon atoms such as, for example, hexanoic acid, octanoic (caprylic), nonanoic (pelargonic), decanoic (capric), undecanoic, lauric, tridecanoic, tetradecanoic (myristic), pentadecanoic, hexadecanoic (palmitic), heptadecanoic, ocadecanoic (stearic), nonadecanoic, hexadecanoic (palmitic), heptadecanoic, ocatdecanoic (stearic) nonadecanoic, eicosanoic, heneicosanoic, docosanoic and lignoceric acid. Examples of unsaturated carboxylic acids include, but are not limited to, oleic, linoleic and linolenic acid. The term "oil" as used herein, does not include hydrocarbons containing one or more benzene groups or a mixture thereof (e.g. C₉-C₁₀ dialkyl and trialkylbenzenes; SOLVESSO® 100, ExxonMobil Corp., Irving, TX, US).

The expression "pharmaceutically acceptable oil" as used herein, refers to an oil or a mixture thereof suitable for use in veterinary medicine.

The terms "prevent", "preventing" and "prevention" as used herein, refer to inhibiting the inception, or decreasing the occurrence or recurrence, of a disease in an animal subject. Prevention may be complete (e.g. the total absence of pathological cells in the subject) or partial. Prevention also refers to a reduced susceptibility to a clinical condition.

The term "therapeutically effective amount" as used herein, refers to any amount of a composition which, when administered to an animal subject: i) prevents the inception or recurrence or ii) causes the reduction or remission of the disease or condition against which the composition is effective.

The term "treat" or "treatment" as used herein, refers to the administration of a composition of the invention to an animal subject in order to control the progression of a disease after its clinical signs have shown. Control of the progression of a disease is understood to mean the beneficial or desired clinical results that include, but are not limited to, a reduction of the symptoms, a reduction in the duration of the disease, stabilizing or improving a pathological state (specifically to avoid additional deterioration), delaying the progression of the disease and remission (both partial and total). Control of the progression of a disease also involves an extension of the survival of an animal subject, compared with the expected survival of the subject if treatment is not applied.

### 2. Topical liquid compositions

The non-aqueous topical composition of the present invention comprises:
i) moxidectin or its pharmaceutically acceptable salts,
ii) from about 1% to 50% (w/v) of isopropyl alcohol,
iii) from about 1% to 20% (w/v) of at least one thickener, and
iv) a pharmaceutically acceptable oil.

In one embodiment, the composition comprises from about 0.001% to 10% (w/v) moxidectin or its pharmaceutically acceptable salts, based on the total volume of the composition. Preferably, the composition comprises from about 0.01% to 5% (w/v) of moxidectin or its pharmaceutically acceptable salts. Compositions comprising from about 0.5% or 2.5% (w/v) of moxidectin or its pharmaceutically acceptable salts are most preferred.

In another embodiment, the composition comprises from about 1% to 40% (w/v) of isopropyl alcohol, based on the total volume of the composition. Preferably, the composition comprises from about 1% to 20% (w/v) of isopropyl alcohol. More preferably, the composition comprises from about 5% to 15% (w/v) or from about 7.5% to 12.5% (w/v) of isopropyl alcohol. Most preferably, the composition comprises from about 10% (w/v) of isopropyl alcohol.

In an additional embodiment, the composition further comprises at least one penetration enhancer. A penetration enhancer is a substance that modifies the permeability of connective tissue, and thus, facilitates the passage of moxidectin through the skin. Penetration enhancers constitute various classes of compounds that include, but are not limited to, fatty acids or alcohols (e.g. linoleic acid, oleic acid, oleyl alcohol), certain fatty acid esters, lipophilic compounds (e.g. laurocapram) and certain solvents (e.g. dimethylacetamide, DMSO, ethanol, ethyl acetate, propylene glycol, pyrrolidones and other compounds that are capable of disrupting the barrier function of the *stratum corneum)* or a mixture thereof. Penetration enhancers can also act as spreading agents.

Preferably, the penetration enhancer is a fatty acid ester comprising isopropyl myristate, methyl caprate, methyl noanoate, a polyethylene (PEG) glycol ester, a polypropylene glycol (PPG) ester or a mixture thereof. More preferably, the penetration enhancer is a PPG ester. Most preferably, the PPG ester is PPG-2 myristyl ether propionate (e.g. CRODAMOL® PMP, Croda International P1c, East Yorkshire, GB). *See* Walters K, Ed., "Dermatological and Transdermal Formulations" (CRC Press, Boca Raton, FL, US, 2002; ISBN 978-0-8247-4323-9).

Preferably, the composition comprises from about 1% to 20% (w/v) of the penetration enhancer, based on the total volume of the composition. More preferably, the composition comprises from about 5% to 15% (w/v) or from about 7.5% to 12.5% (w/v) of the penetration enhancer. Most preferably, the composition comprises from about 10% (w/v) of the penetration enhancer.

In a further embodiment, the composition comprises from about 1% to 20% (w/v) of at least one thickener, based on the total volume of the composition. Preferably, the composition comprises from about 5% to 15% (w/v) of the thickener. More preferably, the composition comprises from about 10% (w/v) of the thickener.

A thickener is a substance that can increase the viscosity of a composition without substantially modifying the efficacy of the active agent in the composition. Examples of thickeners include, but are not limited to, carboxylic acid polymers, crosslinked polyacrylate polymers, polyacrylamide polymers, polysaccharides, gums, unsaturated aliphatic polymers or a mixture thereof.

Examples of carboxylic acid polymers include, but are not limited to, crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol. *See* US5087445 and US4509949. Examples of commercially available carboxylic acid polymers include carbomers, which are homopolymers of acrylic acid crosslinked with allyl ethers of sucrose, or pentaerytritol.

Crosslinked polyacrylate polymers include, but are not limited to, cationic and nonionic polymers. *See* US5100660, US4849484, US4835206, US462078 and US4599379.

Polyacrylamide polymers include, but are not limited to, polyacrylamide, isoparaffin and laureth-7, multi-block copolymers of acrylamides and substituted acrylamides with acrylic acids and substituted acrylic acids.

Polysaccharides include, but are not limited to, carboxymethyl hydroxyethylcellulose, cellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate or mixtures thereof. Further examples of polysaccharides that can be used in the invention include, but are not limited to, an alkyl substituted cellulose where the hydroxy groups of the cellulose polymer are hydroxyalkylated, preferably hydroxyethylated or hydroxypropylated, to form a hydroxyalkylated cellulose which is then further modified with a C₁₀-C₃₀ straight chain or branched chain alkyl group through an ether linkage. Typically, these polymers are ethers of C₁₀-C₃₀ straight or branched chain alcohols with hydroxyalkylcelluloses. Other useful polysaccharides include scleroglucans comprising a linear chain of (1-3) linked glucose units with a (1-6) linked glucose every three units.

Gums that can be used with the present invention include, but are not limited to, acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluroinic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboyxmethyl dextran, sodium carrageenan, tragacanth gum, xanthan gum or mixtures thereof.

Unsaturated aliphatic polymers that can be used with the present invention include, but are not limited to, oligomers such as polyethylene, polyisobutylene and polybutene. More preferably, the thickener is a polybutene. Preferably, the polybutene has a number average molecular weight (Mₙ) from about 320 to 2300. More preferably, the polybutene has a Mₙ from about 810 to 1800.

The composition of the invention comprises a pharmaceutically acceptable oil. Suitable oils than can be used with the invention include, but are not limited to, mono-, di- and triglycerides, fatty acids (e.g. oleic, linoleic, palmitic and stearic acid) and their esters, ethers and esters of propylene glycol or other polyols. In certain embodiments, the oil comprises short, medium or long chain triglycerides or a mixture thereof. Preferably, the oil comprises medium chain triglycerides (MCTs). More preferably, the MCTs are C₈-C₁₀ triglycerides or a mixture thereof. Most preferably, the oil comprises caprylic and capric triglycerides or a mixture thereof.

The composition of the invention may also contain one or more excipients including, but not limited to, an antioxidant, a bittering agent, a coloring agent, a base, a preservative, a crystallization inhibitor, a solvent or a mixture thereof. Preferably, the composition also contains an antioxidant. In another embodiment, the composition contains an additional active agent. The excipients and additional active agents should be selected to avoid any chemical incompatibilities with moxidectin and any other ingredients present in the composition.

Antioxidants that can be used with the composition of the present invention include, but are not limited to, acetyl cysteine, ascorbic acid, ascorbyl dipalmitate, ascorbyl methylsilanol pectinate, ascorbyl palmitate, ascorbyl stearate, butylated hydroxyanisole (BHA), tert-butylhydroquinone (TBHQ), citric acid anhydrous, cysteine, cysteine HCl, diamylhydroquinone, di-tert-butylhydroquinone, dicetyl thiodipropionate, dioleyl tocopheryl methylsilanol, disodium ascorbyl sulfate, distearyl thiodipropionate, ditridecyl thiodipropionate, dodecyl gallate, erythorbic acid, esters of ascorbic acid, ethyl ferulate, ferulic acid, gallic acid esters, hydroquinone, isooctyl thioglycolate, kojic acid, magnesium ascorbate, magnesium ascorbyl phosphate, methylsilanol ascorbate, natural botanical antioxidants such as green tea or grape seed extracts, nordihydroguaiaretic acid, octyl gallate, phenylthioglycolic acid, potassium ascorbyl tocopheryl phosphate, potassium sulfite, propyl gallate, quinones, rosmarinic acid, sodium ascorbate, sodium bisulfite, sodium erythorbate, sodium metabisulfite, sodium sulfite, superoxide dismutase, sodium thioglycolate, sorbityl furfural, thiodiglycol, thiodiglycolamide, thiodiglycolic acid, thioglycolic acid, thiolactic acid, thiosalicylic acid, tocophereth-5, tocophereth-10, tocophereth-12, tocophereth-18, tocophereth-50, tocopherol, tocophersolan, tocopheryl acetate, tocopheryl linoleate, tocopheryl nicotinate, tocopheryl succinate, tris(nonylphenyl)phosphite and mixtures thereof. Preferably, the antioxidant comprises BHA, TBHQ, citric acid anhydrous or a mixture thereof.

The antioxidant can be present in an amount of from about 0.0001% (w/v) to 1% (w/v), based on the total volume of the composition. Preferably, the antioxidant is present in an amount of from about 0.001% (w/v) to 0.5% (w/v). More preferably, the antioxidant is present in an amount of from about 0.01% (w/v) to 0.05% (w/v).

The composition may also contain a bittering (e.g. brucine, enatonium benzoate, quassin, quercetin, sucrose octaacetate) or coloring agent (e.g. food blue no. 2, food lake dye, food red no. 3, food yellow no. 5, red iron oxide, titanium dioxide, yellow iron oxide).

A base can also be incorporated into the composition. Examples of suitable bases include, but are not limited to, barium hydroxide, calcium hydroxide, cesium hydroxide, lithium hydroxide, potassium hydroxide, rubidium hydroxide, sodium hydroxide, strontium hydroxide, triethanolamine or a mixture thereof.

Preservatives can also be incorporated into the composition to protect it from microbial contamination. Examples of suitable preservatives include, but are not limited to, benzoic acid, benzyl alcohol, methyl alcohol and their salts, phenolic compounds (e.g. phenol), quaternary compounds (e.g. benzalkonium chloride, butyl parahydroxybenzoate, ethyl parahydroxybenzoate, methyl parahydoxybenzoate, propyl parahydoxybenzoate), parabens (e.g. methyl-, ethyl-, propyl-, butyl- paraben) or a mixture thereof.

The composition may additionally include a crystallization inhibitor. Examples of suitable crystallization inhibitors include, but are not limited to, acrylic derivatives (e.g. methacrylates), alkyl sulphates (e.g. sodium cetyl sulphate, sodium lauryl sulphate), amphoteric surfactants (e.g. lauryl-substituted betaine compounds), anionic surfactants (e.g. ammonium, calcium, potassium, sodium and triethanolamine stearates), benzyl alcohol, cationic surfactants (e.g. cetyltrimethylamtnonium bromide, octadecylamine hydrochloride), fatty acids (e.g. coconut oil derivates), glycerol, lecithin, mannitol, sorbitol or a mixture thereof.

A solvent can also be incorporated into the composition to enhance its fluidity. Examples of solvents include, but are not limited to, acetone, ethanol, glycerol, methanol, n-propyl alcohol, propylene glycol, dipropylene glycol and tripropylene glycol. Preferably, the solvent is propylene glycol.

The composition may also contain other active agents such as a compound member of the benzimidazole (e.g. albendazole, mebendazole, triclabendazole), neonicotinoid (e.g. acetamiprid, clothianidin, imidacloprid, nitenpyram, nithiazine, thiacloprid, thiamethoxam), phenyl pyrazole (e.g. fipronil) families or a mixture thereof. Preferably, the benzimidazole compound is triclabendazole. Preferably, the neonicotinoid compound is imidacloprid.

In yet another embodiment, the non-aqueous topical composition of the invention consists essentially of:
i) moxidectin or its pharmaceutically acceptable salts,
ii) from about 1% to 50% (w/v) of at least one C₁-C₆ alcohol,
iii) from about 1% to 20% (w/v) of at least one penetration enhancer,
iv) from about 1% to 20% (w/v) of at least one thickener,
v) a pharmaceutically acceptable oil and,
vi) optionally, an antioxidant.

The composition of the present invention is a topical composition that can be in pour-on, spot-on or spray-on form. Preferably, the composition is in a pour-on form. Said forms of the composition can be produced following methods known in the art. *See* Gunnar A, Ed., "Remington: The Science and Practice of Pharmacy" 20th ed. (Lippincott Williams & Wilkins, Philadelphia, PA, US, 2003).

In a preferred embodiment, the non-aqueous topical composition of the invention comprises:
i) moxidectin or its pharmaceutically acceptable salts,
ii) from about 1% to 20% (w/v) of a C₁-C₆ alcohol,
iii) from about 1% to 20% (w/v) of a polypropylene glycol ester,
iv) from about 1% to 20% (w/v) of an unsaturated aliphatic polymer, and
v) a liquid phase comprising a C₈-C₁₀ medium chain triglyceride or a mixture thereof.

More preferably, the composition of the invention is in pour-on or spot form and comprises:
(a) from about 0.01-5% (w/v) moxidectin or its pharmaceutically acceptable salts, from about 5-15% (w/v) of a C₁-C₆ alcohol, from about 5-15% (w/v) of a polypropylene glycol ester, from about 5-15% (w/v) of an unsaturated aliphatic polymer and a liquid phase comprising a mixture of C₈-C₁₀ medium chain triglycerides,
(b) from about 0.01-5% (w/v) moxidectin or its pharmaceutically acceptable salts, from about 5-15% (w/v) of a C₁-C₆ alcohol, from about 7.5-12.5% (w/v) of a polypropylene glycol ester, from about 5-15% (w/v) of an unsaturated aliphatic polymer and a liquid phase comprising a mixture of C₈-C₁₀ medium chain triglycerides,
(c) from about 0.01-5% (w/v) moxidectin or its pharmaceutically acceptable salts, from about 5-15% (w/v) of a C₁-C₆ alcohol, from about 5-15% (w/v) of a polypropylene glycol ester, from about 7.5-12.5% (w/v) of an unsaturated aliphatic polymer and a liquid phase comprising a mixture of C₈-C₁₀ medium chain triglycerides,
(d) from about 0.01-5% (w/v) moxidectin or its pharmaceutically acceptable salts, from about 5-15% (w/v) of a C₁-C₆ alcohol, from about 7.5-12.5% (w/v) of a polypropylene glycol ester, from about 7.5-12.5% (w/v) of an unsaturated aliphatic polymer and a liquid phase comprising a mixture of C₈-C₁₀ medium chain triglycerides,
(e) from about 0.01-5% (w/v) moxidectin or its pharmaceutically acceptable salts, from about 7.5-12.5% (w/v) of a C₁-C₆ alcohol, from about 5-15% (w/v) of a polypropylene glycol ester, from about 5-15% (w/v) of an unsaturated aliphatic polymer and a liquid phase comprising a mixture of C₈-C₁₀ medium chain triglycerides,
(f) from about 0.01-5% (w/v) moxidectin or its pharmaceutically acceptable salts, from about 7.5-12.5% (w/v) of a C₁-C₆ alcohol, from about 7.5-12.5% (w/v) of a polypropylene glycol ester, from about 5-15% (w/v) of an unsaturated aliphatic polymer and a liquid phase comprising a mixture of C₈-C₁₀ medium chain triglycerides,
(g) from about 0.01-5% (w/v) moxidectin or its pharmaceutically acceptable salts, from about 7.5-12.5% (w/v) of a C₁-C₆ alcohol, from about 5-15% (w/v) of a polypropylene glycol ester, from about 7.5-12.5% (w/v) of an unsaturated aliphatic polymer and a liquid phase comprising a mixture of C₈-C₁₀ medium chain triglycerides,
(h) from about 0.01-5% (w/v) moxidectin or its pharmaceutically acceptable salts, from about 7.5-12.5% (w/v) of a C₁-C₆ alcohol, from about 7.5-12.5% (w/v) of a polypropylene glycol ester, from about 7.5-12.5% (w/v) of an unsaturated aliphatic polymer and a liquid phase comprising a mixture of C₈-C₁₀ medium chain triglycerides.

### 3. Process of making

In another aspect, the present invention is directed to a process of preparing the non-aqueous topical composition of the invention, which comprises the step of mixing moxidectin or its pharmaceutically acceptable salts, at least one C₁-C₆ alcohol, at least one thickener and a pharmaceutically acceptable oil under a nitrogen-rich oxygen-free atmosphere. In one embodiment of this aspect, at least one penetration enhancer is further added to the composition mixture.

Preferably, moxidectin or its pharmaceutically acceptable salts is mixed with from about 1% to 50% (w/v) of a C₁-C₆ alcohol, a polypropylene glycol ester and an unsaturated aliphatic polymer in the presence of a pharmaceutically acceptable oil to yield the composition of the invention. In an additional version of this aspect, the process of making the composition of the invention further comprises adding at least one antioxidant to the bulk product mixture.

### 4. Use of topical composition for treating or preventing ecto- and endoparasite infestations

In another aspect, the present invention refers to a non-aqueous topical composition according to the invention for use as a medicament. Preferably, the composition is for use in the treatment or prevention of endoparasites, ectoparasites or both in an animal subject. Preferably, the subject is a mammal. More preferably, the subject is a cat, dog, sheep, cow or horse. Preferably, a therapeutically effective amount of the composition is administered to the animal subject.

Parasitic diseases may be caused by either endoparasites or ectoparasites. As used herein endoparasites refer to those parasites living inside the body of the host, either within an organ (e.g. stomach, lungs, heart, intestines) or simply under the skin. Ectoparasites are parasites that live on the outer surface of the host but still draw nutrients from it.

Endoparasitic diseases may be further classified according to the parasite involved in the infection. For example, endoparasitic diseases generally referred to as helminthiasis are due to infections caused by parasitic worms known as helminths. Helminthiasis is a prevalent and serious public health problem involving infection of domesticated animals such as swine, sheep, horses, cattle, goats, dogs, cats and poultry. Many of these infections are caused by a subgroup of worms described as nematodes. The most common genera of nematodes infecting the animals referred to before include, but are not limited to, *Haemonchus spp, Trichostrongylus spp, Ostertagia spp, Nematodirus spp, Cooperia spp, Ascaris spp, Bunostomum spp, Oesophagostomum spp, Chabertia spp, Trichuris spp, Strongylus spp, Trichonema spp, Dictyocaulus spp, Capillaria spp, Heterakis spp, Toxocara spp, Ascaridia spp, Oxyuris spp, Ancylostoma spp, Uncinaria spp, Toxascaris spp* and *Parascaris spp.* Many parasites are specific to a species (i.e. infect only one type of host). Most also have a preferred site of infection within the animal. Thus, *Haemonchus spp* and *Ostertagia spp* primarily infect the stomach, while *Nematodirus spp* and *Cooperia spp* mostly attack the intestines. Other endoparasites reside in the heart, eyes, lungs or blood vessels of a host, while others are subcutaneous parasites. Helminthiasis can lead to weakness, weight loss, anemia, intestinal damage, malnutrition and organ failure. If left untreated, these diseases can result in the death of the animal.

Examples of endoparasites which infect animals include, but are not limited to, gastro-intestinal parasites of the genera *Ancylostoma spp, Necator spp, Ascaris spp, Strongyloides spp, Trichinella spp, Capillaria spp, Trichuris spp* and *Enterobius spp.* Other endoparasites which infect animals are found in the blood or in other organs. Examples of such parasites include, but are not limited to, filarial worms (e.g. *Wuchereria spp, Brugia spp, Onchocerca spp),* as well as extra-intestinal stages of the intestinal worms (e.g. *Strongylides spp, Trichinella spp).* Ectoparasites which infest domestic animals include arthropods (e.g. ticks, fleas, mites, mosquitos, lice). Infections by these parasites can result in serious and even fatal diseases.

Infestations by ectoparasitic arthropods can also interfere with normal eating habits, thus causing weight loss in an animal subject. Ectoparasitic infestations may give rise to serious diseases including, but not limited to, encephalitis, anaplasmosis, babesiosis, Rocky Mountain spotted fever, Lyme disease, ehrlichiosis, West Nile virus, swine pox, malaria and yellow fever, many of which can be fatal to the host. Animals may be infected by several species of parasite at the same time, since infection by one parasite may weaken the animal and make it more susceptible to infection by a second species of parasite.

In one embodiment, the composition of the invention is used for treating or preventing endoparasites, ectoparasites or both in a cow. Preferably, the composition of the invention is used for treating or preventing parasitic infestations caused by gastrointestinal roundworms (e.g. *Ostertagia ostertagi, O. radiatum, Haemonchus placei, Trichostrongylus axei, T. colubriformis, Cooperia oncophora, C. pectinata, C. punctata, C. spatulata, C. surnabada, Bunostomum phlebotomum, Oesophagostomum radiatum, Nematodirus helvetianus),* lungworms (e.g. *Dictyocaulus viviparus),* cattle grubs (e.g. *Hypoderma bovis, H. lineatum*)*,* mites (e.g. *Chorioptes bovis, Psoroptes ovis (P. communis var. bovis),* lice (e.g. *Linognathus vituli, Haematopinus eurysternus, Solenopotes capillatus, Bovicola (Damalinia) bovis)* and horn flies (e.g. *Haematobia irritans)* in a cow.

Preferably, the composition of the invention is administered periodically, when applied to a cow. The dosage regimen may vary according to the clinical protocols that are usually applicable to cattle as known in the art. Preferably, the composition is administered topically at a minimum of 0.5 mg moxidectin per kilogram of body weight when applied to a cow.

In one embodiment, the composition of the invention is used for treating or preventing endoparasites, ectoparasites or both in a dog. Preferably, the composition of the invention is used for treating or preventing parasitic infestations caused by heartworms (e.g. *Dirofilaria immitis),* intestinal hookworms (e.g. *Ancylostoma caninum, Uncinaria stenocephala),* roundworms (e.g. *Toxocara canis, Toxascaris leonina)* and whipworm (e.g. *Trichuris vulpis)* in dogs.

Preferably, the composition of the invention is administered periodically, such as once monthly, when applied to a dog. The dosage regimen may vary according to the clinical protocols that are usually applicable to dogs as known in the art. Preferably, the composition is administered topically at a minimum of 2.5 mg moxidectin per kilogram of body weight when applied to a dog.

The composition of the invention may be applied at one, two or more points in an animal subject. Preferably, the composition is applied to the area localized between the animal's shoulders. The composition may also be applied in a continuous pouring action from a point between the animal's shoulders to a point near the base of its tail.

This invention is further illustrated by the following examples .

### Example 1

### Process of making

A composition according to the present invention was prepared by first adding 2 kg of medium-chain triglycerides (NFOBFF® M-5, Stepan Specialty Products, LLC, Maywood, NJ, US) and 40 kg of polybutene (INDOPOL® H-300, Ineos Group Ltd., Rolle, CH) to a manufacturing vessel. The mixture was then blended using an agitator mixer operated at low speed for 15 minutes. Secondly, 40 kg of isopropyl alcohol was added to the manufacturing vessel and was blended using an agitator mixer operated at low speed for 15 minutes. Thirdly, 40 kg of PPG-2 myristyl ether propionate (CRODAMOL® PMP, Croda International Plc, East Yorkshire, GB), 0.012 kg of tert-butyl hydroquinone (TBHQ), 0.04 kg of butylated hydroxyanisole (BHA), 0.008 kg of citric acid anhydrous and 0.14 kg of propylene glycol were added to the manufacturing vessel. The mixture was then blended using an agitator mixer operated at low speed until no air bubbles were noticeable in the bulk product. Fourthly, 2 kg of moxidectin was added to the manufacturing vessel and was blended using a silverson mixer and an agitator mixer operated at low speed for 20 minutes. Finally, medium-chain triglycerides (NEOBEE® M-5, Stepan Specialty Products, LLC, Maywood, NJ, US) were added to the manufacturing vessel until a total volume of 400 L was completed. The mixture was then blended using a silverson mixer and an agitator mixer operated at low speed for 15 minutes to yield the Moxip-010 formulation. All the steps in the process were conducted in a blanket vessel with oxygen-free nitrogen. Aliquots of the Moxip-010 formulation were prepared and stored until use. Table 1 depicts the quantitative composition of the Moxip-010 formulation.

**Table 1**

| Composition of the Moxip-010 formulation | | |
|---|---|---|
| Ingredients | % w/v | mg/mL |
| Moxidectin | 0.5 | 5 |
| Isopropyl alcohol | 10 | 100 |
| Polybutene (Indopol® H-300) | 10 | 100 |
| PPG-2 Myristyl ether propionate (Crodamol® PMP) | 10 | 100 |
| tert-Butyl hydroquinone (TBHQ) | 0.003 | 0.03 |
| Butylated hydroxyanisole (BHA) | 0.01 | 0.1 |
| Citric acid anhydrous | 0.002 | 0.02 |
| Propylene glycol | 0.035 | 0.35 |
| Medium-chain triglycerides (Neobee® M-5 oil) | to 100% v/v | to 1 mL |

### Example 2

### Stability analysis

A comparative stability analysis was conducted between the Moxip-010 formulation of Example 1 and Cydectin® 0.5% (w/v) pour-on for cattle (Zoetis UK Ltd, Vm:42058/4025), a commercially available moxidectin pour-on formulation. The Cydectin® reference product has a 24-month shelf life.

The study was performed at (i) 40°C ± 2°C and 75% ± 5% relative humidity (RH) for 6 months and (ii) 30°C ± 2°C and 65% ± 5% RH for 12 months. *See* Tables 2 and 3. An additional stability test was carried out with the Moxip-010 formulation only at 25°C ± 2°C and 60% ± 5% RH for 18 and 36 months. *See* Table 4. The levels of known and unknown impurities in the moxidectin formulations were determined according to protocols widely employed in the art. *See* Ph. Eur. 9.0 section 01/2008:1656 (August 2016) and USP 39, Vol. 38(5), pages 4947-4949 (August 2016). The total level of impurities was also evaluated.

The analysis demonstrates that the total level of impurities is lower in the Moxip-010 formulation than in the Cydectin® product at 40°C/75% RH for 6 months and at 30°C/65 RH for 12 months. *See* Tables 2 and 3. The Moxip-010 formulation is at least 4-times purer than the Cydectin® product (i.e. 11.88/2.93=4.05). *See* Table 2. In addition, the results for the 25°C/60% RH test shows that the total level of impurities of the Moxip-010 formulation remain about the same after 18 months (5.44%) and 36 months (5.36%) of storage. Moreover, the level of unknown impurities is from 4 to 10 times lower in the Moxip-010 formulation than in the Cydectin® product. *See* Tables 2 and 3.

The data above proves that the Moxip-010 formulation is more stable than the Cydectin® product. Moreover, the Moxip-010 formulation remains stable for at least a year longer than the Cydectin® product. In addition, the low content of unknown impurities of the Moxip-010 formulation in comparison with the Cydectin® product suggests that the former is a better characterized and more reliable formulation. Finally, the Moxip-010 formulation is also particularly suited for use in areas characterized by warm temperatures and high humidity, such as in the tropical region, because of its enhanced stability.

**Table 2**

| 40°C ± 2°C/75% ± 5% RH @ 6 months | | | |
|---|---|---|---|
| Parameter | Study specification | Moxip-0 10 | Cydectin® |
| (i) Appearance | A translucent, colorless to pale yellow slightly viscous solution. | Complies | Complies |
| (ii) Moxidectin content | 0.45 to 0.525% w/v | 0.47% | 0.46% |
| (iii) Total known and unknown impurities (including degradation products) | Known ≤ 17.7% | 2.00% | 6.74% |
| | Unknown ≤ 12.3% | 0.93% | 5.14% |
| | Total ≤ 30.0% | 2.93% | 11.88% |

**Table 3**

| 30°C ± 2°C/65% ± 5% RH @ 12 months | | | |
|---|---|---|---|
| Parameter | Study specification | Moxip-010 | Cydectin® |
| (i) Appearance | A translucent, colorless to pale yellow slightly viscous solution. | Complies | Complies |
| (ii) Moxidectin content | 0.45 to 0.525% w/v | 0.47% | 0.50% |
| (iii) Total known and unknown impurities (including degradation products) | Known ≤ 17.7% | 2.11% | 6.87% |
| | Unknown ≤ 12.3% | 2.74% | + 23.13% |
| | Total ≤ 30.0% | 4.85% | + 30.00% |

**Table 4**

| 25°C ± 2°C/60% ± 5% RH @ 18 and 36 months | | | |
|---|---|---|---|
| Parameter | Study specification | Moxip-0 10 18 months | Moxip-0 10 36 months |
| (i) Appearance | A translucent, colorless to pale yellow slightly viscous solution. | Complies | Complies |
| (ii) Moxidectin content | 0.45 to 0.525% w/v | 0.45% | 0.46% |
| (iii) Total known and unknown impurities (including degradation products) | Known ≤ 17.7% | 1.63% | 1.42% |
| | Unknown ≤ 12.3% | 3.81% | 3.94% |
| | Total ≤ 30.0% | 5.44% | 5.36% |

## Claims

1. A non-aqueous topical composition comprising:
i) moxidectin or its pharmaceutically acceptable salts,
ii) from 1% to 50% (w/v) of isopropyl alcohol,
iii) from 1% to 20% (w/v) of at least one thickener, and
iv) a pharmaceutically acceptable oil.

2. The composition according to claim 1, comprising from 0.001% to 10% (w/v) moxidectin or its pharmaceutically acceptable salts.

3. The composition according to any one of the preceding claims, comprising from 7.5% to 12.5% (w/v) of the isopropyl alcohol.

4. The composition according to any one of the preceding claims, further comprising from 5% to 15% (w/v) of at least one penetration enhancer.

5. The composition according to any one of the preceding claims, wherein the penetration enhancer is a polypropylene glycol ester.

6. The composition according to claim 5, wherein the polypropylene glycol ester is PPG-2 myristyl ether propionate.

7. The composition according to any one of the preceding claims, wherein the at least one thickener comprises from 5% to 15% (w/v) of an unsaturated aliphatic polymer.

8. The composition according to claim 7, wherein the unsaturated aliphatic polymer is a polybutene.

9. The composition according to claim 8, wherein the polybutene has a number average molecular weight of from 320 to 2300.

10. The composition according to any one of the preceding claims, wherein the pharmaceutically acceptable oil comprises C₈-C₁₀ medium chain triglycerides.

11. The composition according to any one of the preceding claims, wherein the pharmaceutically acceptable oil comprises a mixture of caprylic and capric triglycerides.

12. The composition according to any one of the preceding claims, further comprising an antioxidant, optionally wherein the antioxidant comprises tert-butyl hydroquinone, butylated hydroxyanisole, citric acid anhydrous or a mixture thereof.

13. The composition according to any one of the preceding claims, for use as a medicament.

14. The composition according to any one of claims 1-12, for use in the treatment or prevention of endoparasites, ectoparasites or both in an animal subject.

15. A process of making the composition according to claim 1, which comprises the step of mixing moxidectin or its pharmaceutically acceptable salts, isopropyl alcohol, at least one thickener and a pharmaceutically acceptable oil in a nitrogen-rich oxygen-free atmosphere.

## Patentansprüche

1. Nichtwässrige topische Zusammensetzung, umfassend:
i) Moxidectin oder seine pharmazeutisch verträglichen Salze,
ii) 1 % bis 50 % (Gewicht/Volumen) Isopropylalkohol,
iii) 1 % bis 20 % (Gewicht/Volumen) von zumindest einem Verdickungsmittel, und
iv) ein pharmazeutisch verträgliches Öl.

2. Zusammensetzung nach Anspruch 1, umfassend 0,001 % bis 10 % (Gewicht/Volumen) Moxidectin oder seine pharmazeutisch verträglichen Salze.

3. Zusammensetzung nach einem der vorhergehenden Ansprüche, umfassend 7,5 % bis 12,5 % (Gewicht/Volumen) des Isopropylalkohols.

4. Zusammensetzung nach einem der vorhergehenden Ansprüche, ferner umfassend 5 % bis 15 % (Gewicht/Volumen) von zumindest einem Penetrationsverstärker.

5. Zusammensetzung nach einem der vorhergehenden Ansprüche, wobei der Penetrationsverstärker ein Polypropylenglykolester ist.

6. Zusammensetzung nach Anspruch 5, wobei der Polypropylenglykolester PPG-2-Myristyletherpropionat ist.

7. Zusammensetzung nach einem der vorhergehenden Ansprüche, wobei das zumindest eine Verdickungsmittel 5 % bis 15 % (Gewicht/Volumen) eines ungesättigten aliphatischen Polymers umfasst.

8. Zusammensetzung nach Anspruch 7, wobei das ungesättigte aliphatische Polymer ein Polybuten ist.

9. Zusammensetzung nach Anspruch 8, wobei das Polybuten ein zahlenmittleres Molekulargewicht von 320 bis 2300 aufweist.

10. Zusammensetzung nach einem der vorhergehenden Ansprüche, wobei das pharmazeutisch verträgliche Öl mittelkettige C₈-C₁₀-Triglyceride umfasst.

11. Zusammensetzung nach einem der vorhergehenden Ansprüche, wobei das pharmazeutisch verträgliche Öl eine Mischung aus Capryl- und Capric-Triglyceriden umfasst.

12. Zusammensetzung nach einem der vorhergehenden Ansprüche, ferner umfassend ein Antioxidationsmittel, wobei optional das Antioxidationsmittel tert-Butylhydrochinon, butyliertes Hydroxyanisol, wasserfreie Zitronensäure oder eine Mischung davon umfasst.

13. Zusammensetzung nach einem der vorhergehenden Ansprüche zur Verwendung als Medikament.

14. Zusammensetzung nach einem der Ansprüche 1-12 zur Verwendung bei der Behandlung oder Prävention von Endoparasiten, Ektoparasiten oder beidem bei einem tierischen Subjekt.

15. Verfahren zur Herstellung der Zusammensetzung nach Anspruch 1, das den Schritt des Mischens von Moxidectin oder seinen pharmazeutisch verträglichen Salzen, Isopropylalkohol, zumindest einem Verdickungsmittel und einem pharmazeutisch verträglichen Öl in einer stickstoffreichen sauerstofffreien Atmosphäre umfasst.

## Revendications

1. Composition topique non aqueuse comprenant :
i) de la moxidectine ou ses sels pharmaceutiquement acceptables,
ii) de 1 % à 50 % (p/v) d'alcool isopropylique,
iii) de 1 % à 20 % (p/v) d'au moins un épaississant, et
iv) une huile pharmaceutiquement acceptable.

2. Composition selon la revendication 1, comprenant de 0,001 % à 10 % (p/v) de moxidectine ou de ses sels pharmaceutiquement acceptables.

3. Composition selon l'une quelconque des revendications précédentes, comprenant de 7,5 % à 12,5 % (p/v) d'alcool isopropylique.

4. Composition selon l'une quelconque des revendications précédentes, comprenant en outre de 5 % à 15 % (p/v) d'au moins un activateur de pénétration.

5. Composition selon l'une quelconque des revendications précédentes, dans laquelle l'activateur de pénétration est un ester de polypropylène glycol.

6. Composition selon la revendication 5, dans laquelle l'ester de polypropylène glycol est le PPG-2 myristyl éther propionate.

7. Composition selon l'une quelconque des revendications précédentes, dans laquelle l'au moins un épaississant comprend de 5 % à 15 % (p/v) d'un polymère aliphatique insaturé.

8. Composition selon la revendication 7, dans laquelle le polymère aliphatique insaturé est un polybutène.

9. Composition selon la revendication 8, dans laquelle le polybutène a un poids moléculaire moyen en nombre de 320 à 2 300.

10. Composition selon l'une quelconque des revendications précédentes, dans laquelle l'huile pharmaceutiquement acceptable comprend des triglycérides à chaîne moyenne en C₈ à C₁₀.

11. Composition selon l'une quelconque des revendications précédentes, dans laquelle l'huile pharmaceutiquement acceptable comprend un mélange de triglycérides capryliques et capriques.

12. Composition selon l'une quelconque des revendications précédentes, comprenant en outre un antioxydant, facultativement dans laquelle l' antioxydant comprend de l'hydroquinone de tert-butyle, de l'hydroxyanisole butylé, de l'acide citrique anhydre ou un mélange de ceux-ci.

13. Composition selon l'une quelconque des revendications précédentes, destinée à être utilisée comme un médicament.

14. Composition selon l'une quelconque des revendications 1 à 12, destinée à être utilisée dans le traitement ou la prévention des endoparasites, des ectoparasites ou des deux chez un sujet animal.

15. Procédé de fabrication de la composition selon la revendication 1, qui comprend l'étape de mélange de moxidectine ou de ses sels pharmaceutiquement acceptables, d'alcool isopropylique, d'au moins un épaississant et d'une huile pharmaceutiquement acceptable dans une atmosphère exempte d'oxygène et riche en azote.
